(19) Europäisches Patentamt
European Patent Office
Office européen des brevets

(11) EP 0 344 675 B2

(12) NEW EUROPEAN PATENT SPECIFICATION

(45) Date of publication and mention of the opposition decision:
**11.11.1998 Bulletin 1998/46**

(45) Mention of the grant of the patent:
**18.08.1993 Bulletin 1993/33**

(21) Application number: **89109635.6**

(22) Date of filing: **29.05.1989**

(51) Int. Cl.$^6$: **C07C 209/08**, C07C 209/12, C07C 211/23, C07C 211/27

(54) **Method for the production of selegiline hydrochloride**

Verfahren zur Herstellung von Selegilinhydrochlorid

Procédé pour la préparation de chlorhydrate de sélégiline

(84) Designated Contracting States:
**CH DE GB LI**

(30) Priority: **30.05.1988 CS 3707/88**

(43) Date of publication of application:
**06.12.1989 Bulletin 1989/49**

(73) Proprietor: **FARMAK a.s.**
**771 17 Olomouc (CS)**

(72) Inventors:
- **Hájicek, Josef, Dipl.-Ing. CSc**
  **Praha 2 (CS)**
- **Hrbata, Jiri, PhMr Dr.**
  **Olomouc (CS)**
- **Pihera, Pavel**
  **Praha 10 (CS)**
- **Brunová, Bohumila, Dipl.-Ing.**
  **Praha 5 (CS)**
- **Ferenc, Milan, RNDr**
  **Olomouc (CS)**
- **Krepelka, Jiri, Dipl.-Ing., CSc**
  **Praha 10 (CS)**
- **Kvapil, Lubomir, RNDr**
  **Drahanovice (CS)**
- **Pospisil, Josef**
  **Olomouc (CS)**

(74) Representative:
**Beetz & Partner**
**Patentanwälte**
**Steinsdorfstrasse 10**
**80538 München (DE)**

(56) References cited:
**EP-A- 0 099 302**
**WO-A-88/04552**
**DE-A- 1 568 277**

Remarks:
The file contains technical information submitted after the application was filed and not included in this specification

EP 0 344 675 B2

## Description

The present invention relates to a process for the preparation and industrial production of selegiline hydrochloride (N-methyl-N-(1-phenyl-2-propyl)-2-propinylamine-hydrochloride) of formula I

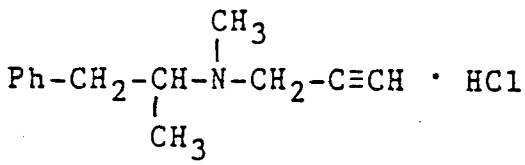

I

wherein Ph is phenyl.

The salt of formula I is a known substance and, especially in the form of its (R)-(-)-enantiomer, a selective MAO B inhibitor, is used in the treatment of the Parkinson disease.

Different methods for the preparation of selegiline starting with 2-methylamino-1-phenylpropane of formula II

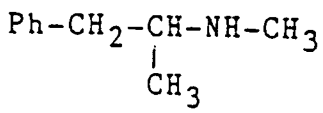

II

wherein Ph is phenyl,
which is propargylated by various methods, are described heretofore in the pertinent literature.

According to one of the prior art processes (GB-A-1031425, AT-A-271437) the above-mentioned starting compound is reacted with dibromopropene at 100 °C for e.g. 7 hours, and the so formed intermediate is subsequently dehydrobrominated and isomerized by the action of a boiling solution of potassium hydroxide for e.g. 16 hours. This procedure is rather laborious and time-consuming from the technological point of view and not suitable for a large-scale production.

In another known process (NL-A-6605956, AT-A-251560), the starting compound 2-methylamino-1-phenylpropane is reacted with propargyl aldehyde, and the intermediary product is then subjected to reduction with aluminum amalgam; serious disadvantages consist in the unstability and the strong irritative properties of propargyl aldehyde.

According to another method (AT-A-252901) the starting compound is propargylated by reaction with acetylene in the presence of formaldehyde and cuprous chloride in dioxane at elevated temperatures for e.g. 30 hours. This procedure is disadvantageous in view of the very long reaction time and the acetylene handling risks.

A technologically more feasible route (GB-A-1031425, DE-B-1227447) is based on the alkylation of N-methylpropargylamine with 2-chloro-1-phenylpropane; in this case, the principal disadvantages consist in the low yields of the desired product and in the expensiveness (high costs) of the starting compound N-methylpropargylamine.

Still other known procedures for the preparation of selegiline comprise the alkylation of 2-methylamino-1-phenylpropane with propargyl bromide at high temperatures either in toluene (NL-A-6605956, US-A-3496195, GB-A-1031425, AT-A-271437) in the same solvent in the presence of lithium amide (CH-A-393306) or in a two-phase binary system of benzene and an aqueous sodium hydroxide solution at temperatures of about 65 °C (EP-A-99302, HU-A-35630). The common disadvantages of these last-mentioned procedures are to be seen in the technological difficulties in connection with the processing of some chemicals at high temperatures and especially the thermal unstability and easy polymerization of propargyl bromide, particularly in the presence of strong alkali.

The selegiline base is converted into its hydrochloride by reaction with hydrogen chloride in ethanol (AT-A-271437), in a benzene - ethanol mixture (EP-A-99302) or in ether (Fowler, J.S., J. Org. Chem. 42, (1977), 2637).

It is the object of the present invention to provide an economical method for the preparation of selegiline hydrochloride yielding the desired product in high purity and with high yields without application of drastic reaction conditions.

The above object is achieved according to claim 1. The dependent claims relate to preferred embodiments.

The process of the present invention consists in the reaction of 2-methylamino-1-phenylpropane (A) with potassium carbonate (B) and propargyl bromide (C) in molar ratios B:A and also C:A of at least 1.1 in an inert organic solvent at a temperature ranging from 5 °C to the boiling temperature of the reaction mixture; the formed base is subsequently converted into the hydrochloride with hydrogen chloride in isopropanol (2-propanol). Suitable organic solvents for the alkylation are chlorinated aliphatic hydrocarbons, particularly chloroform, methylene chloride or dichloroethane, and lower alcohols, e.g. methanol and ethanol, and also dimethylformamide. Accordingly, the reactants B and C are used in an excess of at least 10 %, based on the molar amount of compound A.

Potassium carbonate has a two-fold role in the reaction: It does not only bind the liberated hydrogen bromide, but it substantially increases simultaneously in combination with the inert organic solvent used the nucleophility of the starting compound 2-methylamino-1-phenylpropane, thus facilitating the alkylation. It has been found that apparently similar alkaline agents, e.g. sodium carbonate or calcium carbonate, fail to give equivalent effects. It is also quite surprising that solvent combinations, e.g. with aromatic hydrocarbons, result in a considerable lowering of the reaction yields. Furthermore, the use of isopropanol as the reaction medium for preparing the hydrochloride is particularly advantageous, because in this case the crystallization of the salt proceeds with high yield, and further this particular solvent has a very high purifying effect so that a highly pure material can be obtained.

Suitable starting materials for the alkylation (propargylation) are both the racemic 2-methylamino-1-phenylpropane and either of its optically active chiral isomers.

The preparative route according to the invention thus obviates the use of drastic reaction conditions or hazardous chemicals and provides the desired substance in comparatively high yields and with excellent purity grade.

Further particulars of the procedure of the present invention are illustrated in the following examples.

Example 1

A solution of 20.0 g (0.108 mole) of (R)-(-)-deoxyephedrine hydrochloride in 70 ml of water is added under cooling to 24 ml of a 5N sodium hydroxide solution and 80 ml of chloroform. After shaking the organic phase is separated, and the aqueous phase is extracted with 10 ml of chloroform. The combined chloroform extracts are dried and filtered into a reaction vessel with a charge of anhydrous potassium carbonate (24.5 g, 0.177 mole; 1.64 moles per mole of (R)-(-)-deoxyephedrine hydrochloride), and the drying agent is washed with 10 to 20 ml of chloroform. The stirred heterogeneous mixture is then treated with propargyl bromide in an amount of 10.2 ml (0.134 mole; 1.24 moles per mole of (R)-(-)-deoxyephedrine hydrochloride in a single portion or within 5 minutes). The mixture is stirred for altogether 3 hours, initially if required under cooling with water so that the temperature of the reaction mixture does not exceed 30 to 35 °C. After addition of benzene (50 ml), chloroform (10 ml) and water (125 ml) and stirring the organic phase is separated, washed with water (2 x 50 ml ) and extracted twice with 6 to 7 % hydrochloric acid (130 and 45 ml). The combined acidic extracts are washed with benzene (30 ml) and made alkaline with 30 ml of concentrated aqueous ammonia, and the formed oily precipitate is taken into benzene or toluene (100 and 30 ml). The combined organic phases are washed with water (40 and 30 ml), dried and evaporated in vacuo. The residue is dissolved in isopropanol (30 ml), and hydrogen chloride gas is introduced under stirring and cooling until the pH value of the solution is approx. 3. The solution is then inoculated and allowed to crystallize first for 3 hours at 20 °C and after that for 5 hours at 5 °C. The crystals are separated by suction, washed with isopropanol (approx. 10 ml) and optionally dried. The product is dissolved in 40 ml of hot isopropanol and added with 0.2 g of activated carbon, and after short boiling the mixture is filtered. The filtrate is evaporated to a volume of 35 to 40 ml, and the crystallization and isolation of the crystals is conducted under the above conditions to yield 13.7 g (56.6 %) of (R)-(-)-selegiline hydrochloride (I). M.p. 140.5 to 141.5 °C; optical rotation $[\alpha]_D^{20}$ -11.97° (c = 6.5; water); purity after high-performance liquid chromatography (HPCL) 99.9 %.

Example 2

A solution of 14.9 g (0.10 mole) of (S)-(+)-deoxyephedrine of formula II in 85 ml of methanol is added to 15.2 g (0.11 mole) of anhydrous potassium carbonate (molar ratio of potassium carbonate to (S)-(+)-deoxyephedrine 1.1), and 13.1 g (0.11 mole) of propargyl bromide molar ratio of propargyl bromide to (S)-(+)-deoxyephedrine 1.1) is dropped in within 5 minutes under stirring at a temperature of 5 °C. Stirring of the mixture is continued at this temperature over 24 hours. Thereafter it is diluted with 150 ml of toluene or benzene and 125 ml of water. After short agitation and separation of phases the toluene layer is washed with 2 x 75 ml of water, dried over sodium sulfate and evaporated on a rotary vacuum evaporator. The residue is distilled under reduced pressure (water pump); the fraction boiling in the range of from 126 to 129 °C is collected. The yield is 12.23 g of the base, which is converted into the hydrochloride with use of hydrogen chloride in isopropanol by the procedure of example 1 to give 11.5 g (51.4 %) of (S)-(+)-selegiline hydrochloride (I). M.p. 139.5 to 141 °C; optical rotation $[\alpha]_D^{21}$ + 11.95° (c = 6.3; water); purity after HPLC 99.9 %.

Example 3

The procedure of example 2 is repeated with the only difference that dimethylformamide is used in place of methanol, and the reaction is carried out at a temperature of 30 °C over a period of 2.5 hours. (R)-(-)-deoxyephedrine of formula II thus gives (R)-(-)-selegiline hydrochloride of formula I in a yield of 51.7 %; the physico-chemical properties of the product are the same as those of the product of example 1.

Example 4

The procedure of example 1 is repeated with the difference that methylene chloride is used in place of chloroform, and the reaction with propargyl bromide is performed at the boiling temperature of the mixture. Racemic deoxyephedrine hydrochloride of formula II.HCl thus gives (±)-selegiline hydrochloride of formula ; in a yield of 52.6 %; m.p. of the product 130 to 131.5 °C.

Examples 5 and 6

The procedure of example 1 is repeated, and the resulting reaction mixture is processed as in example 2 with the difference that dichloroethane (example 5) and ethanol (example 6) are used in place of chloroform. (R)-(-)-deoxyephedrine (II) so gives (R)-(-)-selegiline hydrochloride (I) in a yield of 47.8 % (example 5) and 49.2 % (example 6), the properties of the product corresponding to those of the product of example 1.

## Claims

1. A method for preparing selegiline hydrochloride of formula I

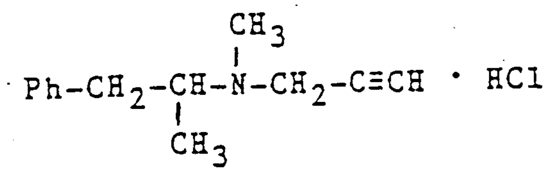

(I),

wherein Ph is phenyl, by

(1) propargylation of 2-methylamino-1-phenylpropane of formula II

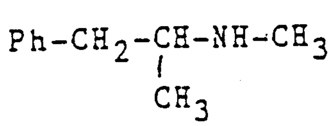

(II),

wherein Ph is phenyl,
with propargyl bromide in the presence of an inert organic solvent and an alkali,
and
(2) conversion of the obtained base with hydrogen chloride in an organic solvent into the hydrochloride,

characterized in that

- in step 1, the 2-methylamino-1-phenylpropane (A) of formula II is allowed to react in the inert organic solvent with the propargyl bromide (C) in the presence of anhydrous potassium carbonate (B) in a molar ratio B:A and also C:A of ≥ 1.1 at a temperature from 5 °C to the boiling temperature of the reaction mixture, and
- in step 2, isopropanol is used as organic solvent.

2. The method according to claim 1, characterized in that a chlorinated aliphatic hydrocarbon, a lower alcohol or dimethylformamide are used as inert organic solvent in step 1.

3. The method according to claim 1 or 2, characterized in that trichloromethane, dichloromethane and/or dichloroethane are used as inert organic solvent in step 1.

4. The method according to cam 1 or 2, characterized in that ethanol and/or methanol are used as inert organic solvent in step 1.

5. The method according to one of clams 1 to 4, characterized in that racemic 2-methylamino-1-phenylpropane is

used as starting material.

6. The method according to one of claims 1 to 4, characterized in that an optically active chiral isomer of 2-methylamino-1-phenylpropane is used as starting material.

7. The method according to clam 6, characterized in that (R)-(-)-deoxyephedrine or (R)-(-)-deoxyephedrine hydrochloride is used as starting material for preparing (R)-(-)-selegiline hydrochloride.

8. The method according to clam 6, characterized in that (S)-(+)-deoxyephedrine or (S)-(+)-deoxyephedrine hydrochloride is used as starting material for preparing (S)-(+)-selegiline hydrochloride.

9. The method according to one of clams 1 to 8, characterized in that in step 1, the reactants A and B are used in a molar ratio B:A of 1.64.

10. The method according to one of claims 1 to 9, characterized in that in step 1, the reactants A and C are used in a molar ratio C:A of 1.24.

**Patentansprüche**

1. Verfahren zur Herstellung von Selegilin-hydrochlorid der Formel I

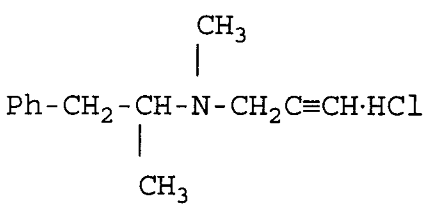

(I),

worin Ph Phenyl darstellt,
durch

(1) Propargylierung von 2-Methylamino-1-phenylpropan der Formel II

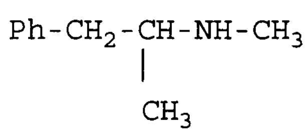

(II),

worin Ph Phenyl darstellt,
mit Propargylbromid in Gegenwart eines inerten organischen Lösungsmittels und einer Alkalie
und

(2) Umwandlung der erhaltenen Base mit Chlorwasserstoff in einem organischen Lösungsmittel in das Hydrochlorid,

dadurch gekennzeichnet, daß

- in Schritt 1 das 2-Methylamino-1-phenylpropan (A) der Formel II im inerten organischen Lösungsmittel mit dem Propargylbromid (C) in Gegenwart von wasserfreiem Kaliumcarbonat (B) in einem Molverhältnis B:A sowie C:A von ≥ 1,1 bei einer Temperatur im Bereich von 5 °C bis zur Siedetemperatur des Reaktionsgemisches umgesetzt
und

- in Schritt 2 als organisches Lösungsmittel Isopropanol verwendet wird.

2. Verfahren nach Anspruch 1, dadurch gekennzeichnet, daß ein chlorierter aliphatischer Kohlenwasserstoff, ein niederer Alkohol oder Dimethylformamid als inertes organisches Lösungsmittel in Schritt 1 verwendet werden.

3. Verfahren nach Anspruch 1 oder 2, dadurch gekennzeichnet, daß Trichlormethan, Dichlormethan und/oder Dichlorethan als organisches Inertlösungsmittel in Schritt 1 verwendet werden.

4. Verfahren nach Anspruch 1 oder 2, dadurch gekennzeichnet, daß Ethanol und/oder Methanol als inertes organisches Lösungsmittel in Schritt 1 verwendet werden.

5. Verfahren nach einem der Ansprüche 1 bis 4, dadurch gekennzeichnet, daß racemisches 2-Methylamino-1-phenylpropan als Ausgangsmaterial verwendet wird.

6. Verfahren nach einem der Ansprüche 1 bis 4, dadurch gekennzeichnet, daß ein optisch aktives chirales Isomer von 2-Methylamino-1-phenylpropan als Ausgangsmaterial verwendet wird.

7. Verfahren nach Anspruch 6, dadurch gekennzeichnet, daß (R)-(-)-Desoxyephedrin oder (R)-(-)-Desoxyephedrin-hydrochlorid als Ausgangsmaterial zur Herstellung von (R)-(-)-Selegilin-hydrochlorid verwendet werden.

8. Verfahren nach Anspruch 6, dadurch gekennzeichnet, daß (S)-(+)-Desoxyephedrin oder (S)-(+)-Desoxyephedrin-hydrochlorid als Ausgangsmaterial zur Herstellung von (S)-(+)-Selegilin-hydrochlorid verwendet werden.

9. Verfahren nach einem der Ansprüche 1 bis 8, dadurch gekennzeichnet, daß in Schritt 1 die Reaktanten A und B in einem Molverhältnis B:A von 1,64 eingesetzt werden.

10. Verfahren nach einem der Ansprüche 1 bis 9, dadurch gekennzeichnet, daß in Schritt 1 die Reaktanten A und C in einem Molverhältnis C:A von 1,24 eingesetzt werden.

**Revendications**

1. Méthode pour préparer le chlorure de sélégiline de la formule I

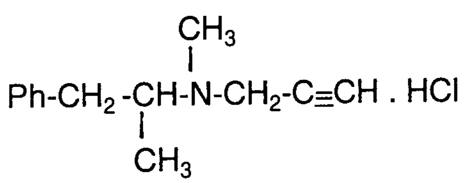

(I)

où Ph est un phényle, par

(1) réaction de propargylation du 2-méthylamino-1-phénylpropane de formule II

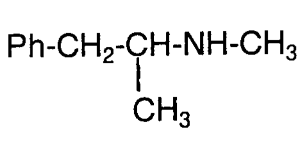

(II)

où Ph est un phényle,
avec du bromure de propargyle en présence d'un solvant organique inerte et d'un alcali, et
(2) conversion de la base obtenue en chlorhydrate avec de l'acide chlorydrique dans un solvant organique,

**caractérisée en ce que**

- dans l'étape 1, on fait réagir le 2-méthylamino-1-phénylpropane (A) de formule (II) dans le solvant organique inerte avec du bromure de propargyle (C) en présence de carbonate de potassium anhydre (B) dans un rapport molaire B:A et également C:A de ≥ 1,1, à une température située entre 5°C et la température d'ébullition

du mélange réactionnel,
et

- dans l'étape 2, de l'isopropanol est utilisé comme solvant organique.

**2.** Méthode selon la revendication 1, caractérisée en ce que l'on utilise un hydrocarbure aliphatique chloré, un alcool inférieur ou le diméthylformamide comme solvant organique inerte dans l'étape 1.

**3.** Méthode selon la revendication 1 ou 2, caractérisée en ce qu'on utilise le trichlorométhane, le dichlorométhane et/ou le dichloroéthane comme solvant organique inerte dans l'étape 1.

**4.** Méthode selon la revendication 1 ou 2, caractérisée en ce qu'on utilise l'éthanol et/ou le méthanol comme solvant organique inerte dans l'étape 1.

**5.** Méthode selon l'une des revendications 1 à 4, caractérisée en ce qu'on utilise le 2-méthylamino-1-phényl-propane racémique en tant que produit de départ.

**6.** Méthode selon l'une des revendications 1 à 4, caractérisée en ce qu'on utilise un isomère chiral optiquement actif du 2-méthylamino-1-phénylpropane comme produit de départ.

**7.** Méthode selon la revendication 6, caractérisée en ce que la (R)-(-)-désoxyéphédrine ou le chlorure de la (R)-(-)-désoxyéphédrine est utilisé comme produit de départ pour préparer le chlorure de (R)-(-)-sélégiline.

**8.** Méthode selon la revendication 6, caractérisée en ce qu'on utilise la (S)-(+)-désoxyéphédrine ou le chlorure de la (S)-(+)-désoxyéphédrine comme produit de départ pour préparer le chlorure de (S)-(+)-sélégiline.

**9.** Méthode selon l'une des revendications 1 à 8, caractérisée en ce que dans l'étape 1, on utilise les réactifs A et B dans un rapport molaire B:A de 1,64.

**10.** Méthode seon l'une des revendications 1 à 9, caractérisée en ce que dans l'étape 1, on utilise les réactifs A et C dans un rapport molaire C:A de 1,24.